# EUROPEAN PATENT SPECIFICATION

(11) **EP 3 166 652 B1**
(45) Date of publication and mention of the grant of the patent: **03.04.2019**
(21) Application number: 14802113.2
(22) Date of filing: 11.07.2014
(51) Int. Cl.: A61L 27/20

(54) **HYALURONIC ACID GELS CROSSLINKED BY SODIUM TRIMETAPHOSPHATE**
DURCH NATRIUMTRIMETAPHOSAT VERNETZTE HYALURONSÄUREGELE
GELS D'ACIDE HYALURONIQUE RÉTICULÉS PAR DU TRIMÉTAPHOSPHATE DE SODIUM

(43) Date of publication of application: 17.05.2017
(73) Proprietor: Allergan Industrie, SAS, 74370 Pringy (FR)
(72) Inventor: PIERRE, Sebastien, 74000 Annecy (FR); LEBRETON, Pierre F., 74000 Annecy (FR)
(74) Representative: Hoffmann Eitle
(86) International application number: PCT/IB2014/001696
(87) International publication number: WO 2016/005785

(56) References cited:
- WO-A1-2014/013286
- WO-A1-2014/181147
- DULONG V ET AL: "Hyaluronan-based hydrogels particles prepared by crosslinking with trisodium trimetaphosphate. Synthesis and characterization", CARBOHYDRATE POLYMERS, APPLIED SCIENCE PUBLISHERS, LTD. BARKING, GB, vol. 57, no. 1, 12 August 2004 (2004-08-12), pages 1-6, XP004522735, ISSN: 0144-8617, DOI: 10.1016/J.CARBPOL.2003.12.006 cited in the application

## Description

### BACKGROUND

The present invention generally relates to a method for preparing injectable compositions for aesthetic use, and more specifically relates to a method for preparing crosslinked hyaluronic acid-based dermal filler compositions as set forth in the claims.

Skin aging is a progressive phenomenon, occurs over time and can be affected by lifestyle factors, such as alcohol consumption, tobacco use and sun exposure. Aging of the facial skin can be characterized by atrophy, slackening, and fattening. Atrophy corresponds to a massive reduction of the thickness of skin tissue. Slackening of the subcutaneous tissues may lead to an excess of skin and ptosis can and lead to the appearance of drooping cheeks and eye lids. Fattening refers to an increase in excess weight by swelling of the bottom of the face and neck. These changes are typically associated with dryness, loss of elasticity, and rough texture.

Hyaluronan, also known as hyaluronic acid (HA) is distributed widely throughout the human body in connective and epithelial tissues and abundant in the different layers of the skin, where it has multiple functions such as, *e*.*g*., to ensure good hydration, to assist in the organization of the extracellular matrix, to act as a filler material, and to participate in tissue repair mechanisms. However, with age, the quantity of HA, collagen, elastin, and other matrix polymers present in the skin decreases. For example, repeated exposure to ultra violet light, *e*.*g*., from the sun, causes dermal cells to both decrease their production of HA as well as increase the rate of their degradation. This HA loss can result in various skin conditions such as, *e*.*g*., imperfects, defects, diseases and/or disorders, and the like. For instance, there is a strong correlation between the water content in the skin and levels of HA in the dermal tissue. As skin ages, the amount and quality of HA in the skin is reduced. These changes lead to drying and wrinkling of the skin.

Dermal fillers are useful in treating soft tissue conditions and in other skin therapies because the fillers can replace lost endogenous matrix polymers, or enhance/facilitate the function of existing matrix polymers, in order to treat these skin conditions. In the past, such compositions have been used in cosmetic applications to fill wrinkles, lines, folds, scars, and to enhance dermal tissue, such as, *e*.*g*., to plump thin lips, or fill-in sunken eyes or shallow cheeks. One common matrix polymer used in dermal filler compositions is HA. Because HA is natural to the human body, it is a generally well tolerated and a fairly low risk treatment for a wide variety of skin conditions. Unfortunately, some HA compositions are less stable to sterilization, such as heat sterilization, than may be desired.

Current HA based dermal fillers are crosslinked with polyepoxides, such as 1,4-butanediol diglycidylether or polyvinylsulfones, such as divinylsulfone or even formaldehyde. WO 2014/013286 A1 describes a dermal filler comprising a crosslinked HA and pyruvate. Dulong et al. (Carbohydrate Polymers 57, pages 1-6) describes HA-based hydrogel microparticles prepared by crosslinking with trisodium trimetaphosphate. WO 2014/181147 A1 describes HA-based dermal fillers prepared by crosslinking with trisodium trimetaphosphate.

The present invention provides a method for preparing new injectable compositions comprising hyaluronic acid crosslinked with (tri)sodium trimetaphosphate. These compositions are monophasic and cohesive hydrogels which can be autoclaved without losing their mechanical properties.

### SUMMARY

The present invention provides a method for preparing compositions, for example, hydrogels, useful as injectable dermal fillers, generally comprising hyaluronic acid crosslinked with (tri)sodium trimetaphosphate (TMP) as defined in the claims.

The compositions are suitable for use as an injectable dermal filler. Further, the compositions are stable to heat sterilization, for example, autoclave sterilization, and they are monophasic and cohesive, exhibiting a smooth and particle-free consistency, and they behave as a single piece when they are injected.

The invention relates to a method of making a monophasic and cohesive composition useful as an injectable dermal filler, the method comprising the steps of crosslinking a hyaluronic acid with TMP.

### DETAILED DESCRIPTION

In accordance with the invention, a crosslinker, such as, (tri)sodium trimetaphosphate (TMP), is added to an uncrosslinked hyaluronic acid to form a crosslinked hyaluronic acid-based composition useful as a dermal filler, for example, for wrinkle filling, volumizing the face, etc.

The present dermal fillers are effective for reducing the appearance of wrinkles in skin, including fine lines, for example, near the eyes, and deep folds, for example, the nasolabial folds. The fillers may be effective for correction of the nasolabial folds, marionette lines, tear troughs, and glabellar frown lines, reduction of the appearance of malar shading, in addition to lip enhancement and cheek augmentation. Other clinical uses may include correction of the jowls and nasal deformities. In general, most patients can expect 6 months of correction, one year of correction, up to 2 years of correction, or longer. The fillers may be injected using a sharp or blunt tipped cannula having a gauge of between 25-27, or as fine as 30 or 32.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups. Unless clearly indicated otherwise, reference to hyaluronic acid, hyaluronan, or HA herein may include its fully protonated, or nonionic form as depicted below, as well as any anionic forms and salts of hyaluronic acid, such as sodium salts, potassium salts, lithium salts, magnesium salts, calcium salts, etc.

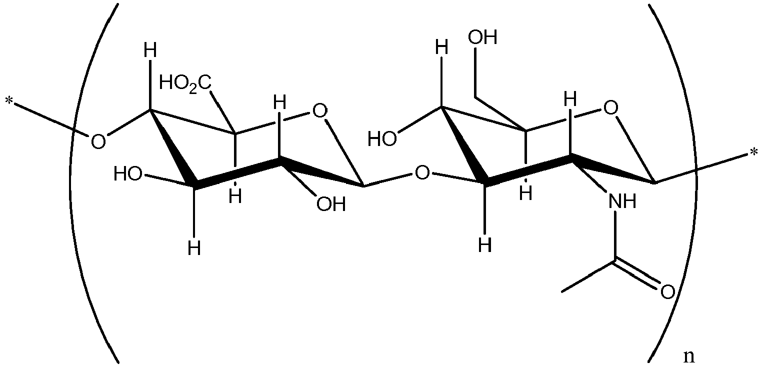

The HA may comprise a mixture of high molecular weight HA, low molecular weight HA, and/or medium molecular weight HA, wherein the high molecular weight HA has an intrinsic viscosity greater than 2.2 L/g, corresponding to a molecular weight greater than about 2,000,000 Da and wherein the low molecular weight HA has an intrinsic viscosity lower than 1.4 L/g, corresponding to a molecular weight of less than about 1,000,000 Da, and the medium molecular weight HA has an intrinsic viscosity between 1.4 L/g and 2.2 L/g, corresponding to a molecular weight of between 1,000,000 Da and 2,000,000 Da. Unless stated otherwise, the molecular weight refers to the weight average molecular weight (Mw).

The weight average molecular weight can be determined using intrinsic viscosity measurement and then conversion to the molecular weight using the Mark-Houvink equation η = 9.78 x 10⁻⁵ x Mw^{0.69} or directly by using size exclusion chromatography against HA of known weight average molecular weight (Mw).

The HA used for crosslinking in the compositions may comprise at least 80% high molecular weight HA, for example, about 90% high molecular weight HA, for example, even 100% high molecular weight HA (all percentages are percentages of total HA weight used for crosslinking).

An uncrosslinked HA fraction may optionally also be included in the compositions, for example, to act as a lubricant and facilitate injection into the skin. Such a composition may comprise an uncrosslinked HA fraction where the added uncrosslinked HA is present at a concentration between 0.1 and 3 mg/g. Preferably, the uncrosslinked HA may be present at a concentration between 0.2 and 1.5 mg/g.

In other embodiments, no uncrosslinked HA is present in the gels, or at least no uncrosslinked HA is added to the gels to act as a lubricant.

Others polysaccharides alternative to, or additional to, hyaluronic acid are contemplated and are considered to be within the scope of the invention. For example, the present invention may comprise a hydrogel composition comprising any suitable glycosaminoglycan polymer. The hydrogel composition disclosed herein can further comprise two or more different glycosaminoglycan polymers. As used herein, the term "glycosaminoglycan" is synonymous with "GAG" and "mucopolysaccharide" and refers to long unbranched polysaccharides consisting of a repeating disaccharide units. The repeating unit consists of a hexose (six-carbon sugar) or a hexuronic acid, linked to a hexosamine (six-carbon sugar containing nitrogen) and pharmaceutically acceptable salts thereof. Members of the GAG family vary in the type of hexosamine, hexose or hexuronic acid unit they contain, such as, e.g., glucuronic acid, iduronic acid, galactose, galactosamine, glucosamine) and may also vary in the geometry of the glycosidic linkage. Any glycosaminoglycan polymer is useful in the hydrogel compositions disclosed herein with the proviso that the glycosaminoglycan polymer improves a condition of the skin when injected or when applied topically to the skin. Non-limiting examples of glycosaminoglycans include chondroitin sulfate, dermatan sulfate, keratan sulfate, hyaluronan. Non-limiting examples of an acceptable salt of a glycosaminoglycans includes sodium salts, potassium salts, magnesium salts, calcium salts, and combinations thereof.

The hydrogel composition may also comprise a crosslinked glycosaminoglycan polymer where the crosslinked glycosaminoglycan polymer is present in an amount sufficient to improve a skin condition. In one aspect of this embodiment, a composition comprises a crosslinked chondroitin sulfate polymer, a crosslinked dermatan sulfate polymer, a crosslinked keratan sulfate polymer, a crosslinked heparan polymer, a crosslinked heparan sulfate polymer, or a crosslinked hyaluronan polymer. In other aspects of this embodiment, a composition comprises a crosslinked glycosaminoglycan where the crosslinked glycosaminoglycan represents, e.g., about 1% by weight, about 2% by weight, about 3% by weight, about 4% by weight, about 5% by weight, about 6% by weight, about 7% by weight, about 8% by weight, or about 9%, or about 10% by weight, of the total glycosaminoglycan present in the composition. In yet other aspects of this embodiment, a composition comprises a crosslinked glycosaminoglycan where the crosslinked glycosaminoglycan represents, e.g., at most 1% by weight, at most 2% by weight, at most 3% by weight, at most 4% by weight, at most 5% by weight, at most 6% by weight, at most 7% by weight, at most 8% by weight, at most 9% by weight, or at most 10% by weight, of the total glycosaminoglycan present in the composition. In still other aspects of this embodiment, a composition comprises a crosslinked glycosaminoglycan where the crosslinked glycosaminoglycan represents, e.g., about 0% to about 20% by weight, about 1% to about 17% by weight, about 3% to about 15% by weight, or about 5% to about 10% by weight, for example, about 11% by weight, about 15% by weight or about 17% by weight, of the total glycosaminoglycan present in the composition.

In aspects of this embodiment, a hydrogel composition comprises a crosslinked glycosaminoglycan where the crosslinked glycosaminoglycan is present at a concentration of, e.g., about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, or about 20 mg/g. In other aspects of this embodiment, a composition comprises a crosslinked glycosaminoglycan where the crosslinked glycosaminoglycan is present at a concentration of, *e*.*g*., at least 1 mg/g, at least 2 mg/g, at least 3 mg/g, at least 4 mg/g, at least 5 mg/g, at least 10 mg/g, at least 15 mg/g, at least 20 mg/g, or at least 25 mg/g, or about 40 mg/g. In yet other aspects of this embodiment, a composition comprises a crosslinked glycosaminoglycan where the crosslinked glycosaminoglycan is present at a concentration of, *e*.*g*., at most 1 mg/g, at most 2 mg/g, at most 3 mg/g, at most 4 mg/g, at most 5 mg/g, at most 10 mg/g, at most 15 mg/g, at most 20 mg/g, at most 25 mg/g, or at most 40 mg/g. In still other aspects of this embodiment, a composition comprises a crosslinked glycosaminoglycan where the crosslinked glycosaminoglycan is present at a concentration of, e.g., about 7.5 mg/g to about 19.5 mg/g, about 8.5 mg/g to about 18.5 mg/g, about 9.5 mg/g to about 17.5 mg/g, about 10.5 mg/g to about 16.5 mg/g, about 11.5 mg/g to about 15.5 mg/g, or about 12.5 mg/g to about 14.5 mg/g, up to about 40 mg/g.

For the crosslinker, the trimetaphosphate salt may comprise sodium trimetaphosphate, calcium trimetaphosphate, barium trimetaphosphate or a trivalent metal cation salt of trimetaphosphate.

The chemical structure of trimetaphosphate salt is shown below:

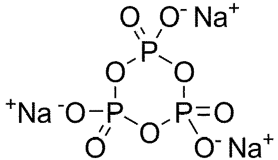

The general chemistry of crosslinking a polysaccharide (ROH) with TMP in an alkaline medium is depicted below, the chemical schematic taken from Lack S., Dulong V. Carbohydrate Research 2007, 342, pp 943-953.

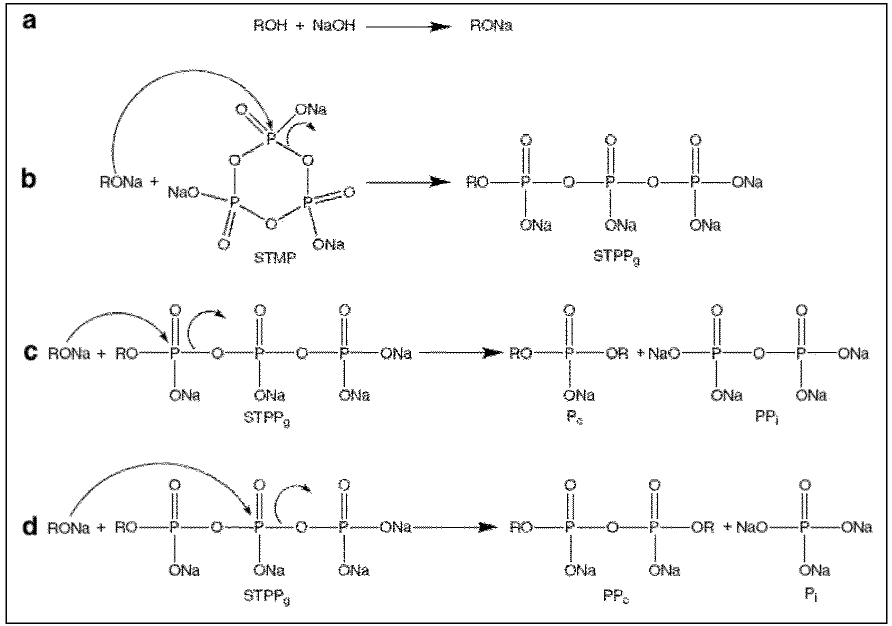

Alternatively, in some embodiments, the crosslinker may comprise metaphosphates other than trimetaphosphate, for example, tetrametaphosphates, pentametaphosphate and mostly hexametaphosphates.

The invention provides an injectable, monophasic hydrogel formulation containing sodium hyaluronate (NaHA) crosslinked with a trimetaphosphate salt. Monophasic as used herein, generally refers to the composition being substantially a single phase gel as opposed to a particulate gel. For example, particulate gels may be described as a gel in which one observes different physical properties in different parts of the gel, i.e. the gel is anisotripic, while monophasic gels may be described as having the same properties throughout, i.e. they are isotropic. Typically, such a formulation, observed under the microscope at a magnification below x90 with a pass-through light set-up, should look smooth, with no or very few visible particles bulging out (see picture 1A for a x85.4 microscopic view of a monophasic hydrogel). Visible particles in such a formulation are considered defects and should represent at most a very minor volumic fraction (less than 0.1%). For example, a gel may be determined to be monophasic or not by use of a dye test. In the dye test, 0.5 g of the gel to be tested is mixed with the same weight of water for an hour by using syringe-to-syringe mixing, and then the mixture is centrifuged in a syringe with an internal diameter under 8 mm to separate the gel from the unabsorbed water. A droplet of water-soluble dye is then deposited on top of the gel and diffusion of the dye through the swollen gel is observed during 10 minutes. The dye is chosen so it is water-soluble but it cannot penetrate hydrogels. Examples of a soluble dye are methylene blue, toluidine blue or eosin. If the dye does not penetrate the swollen gel over 10 minutes, the gel may generally be considered monophasic, meaning no significant (less than 10 wt%, preferably less than 5% or even less than 1%) amount of the initial water was left unabsorbed. If phase separation appears in the swollen gel, the dye will penetrate the liquid phase on top (made primarily of unabsorbed water) and diffuse downwards and the gel will generally be considered not monophasic, for example, biphasic. The same will be observed if the composition contains a significant volumic fraction of visible gel particles in a non-cohesive aqueous matrix (such as water, buffer or uncrosslinked HA). Such materials are neither monophasic, nor cohesive (see an example of non-monophasic and non-cohesive hydrogel in Picture 1B, at x85.4 magnification).

In another aspect of the invention, an injectable, cohesive hydrogel formulation containing sodium hyaluronate (NaHA) crosslinked with a trimetaphosphate salt, is provided. Cohesivity refers to the capacity of the gel to stay attached to itself, for example, meaning the resistance to cutting and the ability to elongate or compress the gel without it separating into pieces. The cohesivity of the gels according to the present invention can be quantified as follows (cf. Derek Jones "Injectable Filers: Principles and Practice, Wiley, 2011, Chapter 3). A small sample of the gel (e.g. 1 mL) is placed onto the plane surface of a rheometer. The sample is placed such that it forms a little heap. A moveable upper plate is placed onto the sample so that the sample is fully covered, i.e. when looking at the plate in a direction perpendicular to the surface of the rheometer, the sample cannot be seen. In order to ensure this, one must chose a plate size that is larger than the sample size. Ideally, the center of the plate is placed over the sample. Typically, for 1mL of gel material, a 25mm diameter upper plate is used.

In the next step of the measurement one then adjusts the gap between the moveable plate and the surface to 2.5 mm. While slowly and steadily moving the plate from this initial position towards a gap width of 0.9 mm within 2 min one records the force (Fn) exerted by the sample in normal direction on the plate.

Once a gap width of 0.9 mm is reached, the system is allowed to relax for 12 minutes. During this time, the measurement is continued. Five measurements are done. To normalize the forces measured, all 5 initial Fn values measured when the test starts are averaged (arithmetic mean) and this resulting average is subtracted from all other data points. The maximum force at the end on the compressive part of this test (when reaching the minimal 0.9mm gap width between the upper plate and the plane) is called the compresson force and is the characteristic value for determining the cohesivity of the gel.

Specifically, a force of 20 gmf (0.1962 N) or more indicates a cohesive material in the sense of the present invention. Gels with lower compression force values are generally not considered cohesive in the context of the present invention. Preferably, the compression force measured as outlined above is at least 25, 30 or 40 gmf. Most preferably, the compression force measured as outlined above is at least 50 or 60 gmf. The accuracy of this measurement is in the order of ±5 gmf.

Such cohesive materials according to the present invention are particularly beneficial in that they can be autoclaved for sterilization also in the presence of other components such as additives, e.g. lidocaine, while retaining mechanical properties which are suited for their use as dermal fillers.

This is most important for a dermal filler as the cohesivity as defined above will contribute to the lift capacity (clinically called the volumizing / bulking effect) provided by the gel clinically, along with its elastic modulus G'.

While cohesive gels can show a good volumizing effect, non-cohesive or weakly cohesive materials with a similar elastic modulus exhibits lower lift capacity due to the non-cohesive gel material spreading more than a more cohesive material when submitted to vertical compression.

To explain the invention further, the formulations described above, since they are cohesive, will behave as giant globular pieces of gel with a volume of at least 0.05 mL (equivalent to a sphere of at least 4.57mm of diameter). When used at similar volumes, the HA microparticles described by Dulong et al. (Carbohydrate Polymers 57(2004)1) do not form monophasic and cohesive gels, since they are much smaller in size and do not stick together, as opposed to the hydrogels described in the invention.

Also, these formulations, when manufactured as dermal fillers, can be made without exposure to the current toxic crosslinkers, without the need of very extensive washings or very long dialysis to remove traces of these toxic crosslinkers.

The level of cohesivity measured as a compression force depends on the final concentration of HA in the formulation, excluding the added uncrosslinked HA. It also depends on several crosslinking, such the type of HA, the pH of the HA during crosslinking, the HA and crosslinker concentrations during crosslinking, the ratio of crosslinker to HA, the time of addition of the crosslinker and the temperature during the crosslinking reaction.

The final HA concentration of the formulations described in this invention should be comprised between 5 and 40 mg/g, preferably between 10 and 30 mg/g, and most preferably between 15 and 25 mg/g.

For the crosslinking step, the crosslinker and the HA are mixed together to form an intimate solid mix prior to dissolution to yield a very homogenous gel after crosslinking.

The pH during crosslinking should be at least 9, preferably at least 11, and most preferably at least 13, by adapting the concentration of the sodium hydroxide solution accordingly.

The HA concentration during crosslinking should be comprised in between 80 and 100 mg/g, and preferably about 90 mg/g. HA will not crosslink significantly below 80 mg/g. The crosslinked HA gel has a tendency to be fragmented into visible particles above 100 mg/g

The TMP concentration, expressed as a weight ratio (or weight percentage) of TMP versus HA should comprised between 1:1 (100 wt%) and 3:1 (300 wt%) to achieve significant crosslinking into a monophasic gel. Preferably, this ratio should be comprised between 1.5:1 (150 wt%) and 2:1 (200 wt%) so the final formulations can be more cohesive.

The temperature of crosslinking should be comprised between 20°C and 70°C, and preferably between 25°C and 50°C. Crosslinking duration is dependent on the chosen temperature to achieve desired crosslinking degree. It can range from 30 minutes to 72 hours. Non-exhaustive examples of crosslinking duration are about 3 hours at 50°C, and about 24 hours at 25°C.

Hydrogel compositions in accordance with the invention may further and optionally comprise another agent or combination of agents that provide a beneficial effect when the composition is administered to an individual. Such beneficial agents include, without limitation, an antioxidant, an anti-itch agent, an anti-cellulite agent, an anti-scarring agent, an anti-inflammatory agent, an anesthetic agent, an anti-irritant agent, a vasoconstrictor, a vasodilator, an anti-hemorrhagic agent like a hemostatic agent or anti-fibrinolytic agent, a desquamating agent, a tensioning agent, an anti-acne agent, a pigmentation agent, an anti-pigmentation agent, or a moisturizing agent.

The hydrogel compositions disclosed herein may optionally comprise an anesthetic agent. An anesthetic agent is preferably a local anesthetic agent, *i.e*., an anesthetic agent that causes a reversible local anesthesia and a loss of nociception, such as, *e*.*g*., aminoamide local anesthetics and aminoester local anesthetics. The amount of an anesthetic agent included in a composition disclosed herein is an amount effective to mitigate pain experienced by an individual upon administration of the composition. As such, the amount of an anesthetic agent included in a composition disclosed in the present specification is between about 0.1% to about 5% by weight of the total composition. Non-limiting examples of anesthetic agents include lidocaine, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, combinations thereof, and salts thereof. Non-limiting examples of aminoester local anesthetics include procaine, chloroprocaine, cocaine, cyclomethycaine, cimethocaine (larocaine), propoxycaine, procaine (novocaine), proparacaine, tetracaine (amethocaine). Non-limiting examples of aminoamide local anesthetics include articaine, bupivacaine, cinchocaine (dibucaine), etidocaine, levobupivacaine, lidocaine (lignocaine), mepivacaine, piperocaine, prilocaine, ropivacaine, and trimecaine. A composition disclosed herein may comprise a single anesthetic agent or a plurality of anesthetic agents. A non-limiting example of a combination local anesthetic is lidocaine/prilocaine (EMLA).

The compositions disclosed herein may comprise an anesthetic agent in an amount of, e.g., about 0.1%, about 0.2%, about 0.3%, about 0.4%, about 0.5%, about 0.6%, about 0.7%, about 0.8% about 0.9%, about 1.0%, about 2.0%, about 3.0%, about 4.0%, about 5.0%, about 6.0%, about 7.0%, about 8.0%, about 9.0%, or about 10% by weight of the total composition. In yet other aspects, a composition disclosed herein comprises an anesthetic agent in an amount of, *e*.*g*., at least 0.1%, at least 0.2%, at least 0.3%, at least 0.4%, at least 0.5%, at least 0.6%, at least 0.7%, at least 0.8% at least 0.9%, at least 1.0%, at least 2.0%, at least 3.0%, at least 4.0%, at least 5.0%, at least 6.0%, at least 7.0%, at least 8.0%, at least 9.0%, or at least 10% by weight of the total composition. In still other aspects, a composition disclosed herein comprises an anesthetic agent in an amount of, *e.g*., at most 0.1%, at most 0.2%, at most 0.3%, at most 0.4%, at most 0.5%, at most 0.6%, at most 0.7%, at most 0.8% at most 0.9%, at most 1.0%, at most 2.0%, at most 3.0%, at most 4.0%, at most 5.0%, at most 6.0%, at most 7.0%, at most 8.0%, at most 9.0%, or at most 10% by weight of the total composition. In further aspects, a composition disclosed herein comprises an anesthetic agent in an amount of, e.g., about 0.1% to about 0.5%, about 0.1% to about 1.0%, about 0.1% to about 2.0%, about 0.1% to about 3.0%, about 0.1% to about 4.0%, about 0.1% to about 5.0%, about 0.2% to about 0.9%, about 0.2% to about 1.0%, about 0.2% to about 2.0%, about 0.5% to about 1.0%, or about 0.5% to about 2.0% by weight of the total composition.

Hydrogel compositions disclosed herein may also optionally comprise an anti-oxidant agent. The amount of an anti-oxidant agent included in a composition disclosed herein is an amount effective to reduce or prevent degradation of a composition disclosed herein, such as, *e*.*g*., enzymatic degradation and/or chemical degradation of the composition. As such, the amount of an anti-oxidant agent included in a composition may be between about 0.001% to about 10% by weight of the total composition. Non-limiting examples of antioxidant agents include a polyol, a flavonoid, a phytoalexin, an ascorbic acid agent, a tocopherol, a tocotrienol, a lipoic acid, a melatonin, a carotenoid, an analog or derivative thereof, and any combination thereof. A composition disclosed herein may comprise a single antioxidant agent or a plurality of antioxidant agents, a retinol, coenzyme, idebenone, allopurinol, gluthation, sodium selenite.

### EXAMPLES

### Preparation of TMP-crosslinked HA gels

### Instruments:

Rheometer RS600 (G85). Oscillation method according to IP08020.

Imperial Loadcell 1000 from Mecmesin (G76) and Versa test column (G73).

Extrusion force method according to IP04175.

pH-meter (G90). pH method according to IP04172.

### Materials:

Trisodium trimetaphosphate and NaHA are readily available from commercial chemical suppliers.

### Conditions of preparation (parameter ranges):

HA concentration during crosslinking step: 80-100 mg/g.

In these specific examples, high molecular weight HA is about 3,000,000 Da, and low molecular weight HA is about 900,000 Da as indicated in the Tables below.

% of Trisodium trimetaphosphate versus HA (for the crosslinking step): 100 to 300% (weight of crosslinker versus weight of HA).

Duration of crosslinking: 30min to 72h.

Temperature of crosslinking: 20°C to 70°C.

Crosslinking pH: 9-14

### Final ranges:

Final HA or NaHA concentration: 5-40 mg/g
final pH range: 6.0-8.0
Extrusion force : 5 - 15N with a 27G needle and a 0.8mL syringe at 12 to 50mm/min
Extrusion force : 5 - 20N with a 30G needle and a 0.8mL syringe at 12 to 50mm/min

### Preparation steps:

### (1) Hydration step

The NaHA and TMP are mechanically blended, homogenized in their solid forms, to achieve an intimate mix, and then this mix is slowly hydrated using mechanical stirring with a 0.25M NaOH solution.

### (2) Crosslinking Step

The mixture in step (1) is placed in a water bath at 50°C for 3H.

### (3) Neutralization step and homogenization step

A mixture of phosphate buffer and HCI is added to the crosslinked gel mixture to neutralize the solution at a pH around 7, to reach a NaHA concentration of about 25 mg/g , 38 mg/g, or 50 mg/g.

### (4) Dialysis step

The gel is then dialyzed against phosphate buffer for about 24H. The NaHA concentration is then adjusted to at least about 20 mg/g, for example, about 23 mg/g , about 24mg/g, about 25 mg/g, for example, up to about 30 mg/g.

### (5) Sterilization step

The gel stored in syringes is then sterilized with an autoclave.

**Table 1**

| Conditions of making various gels using process of Example 1 | | | | |
|---|---|---|---|---|
| Gel number | [NaHA]during crosslinking (mg/g) | HMW HA (%) | LMW HA (%) | Initial TMP content (wt% versus HA) |
| 3 | 90 | 100 | 0 | 100 |
| 4 | 90 | 100 | 0 | 200 |
| 6 | 90 | 80 | 20 | 300 |
| 7 | 90 | 100 | 0 | 300 |
| 10 | 100 | 80 | 20 | 250 |
| 11 | 90 | 100 | 0 | 150 |
| 12 | 90 | 100 | 0 | 200 |

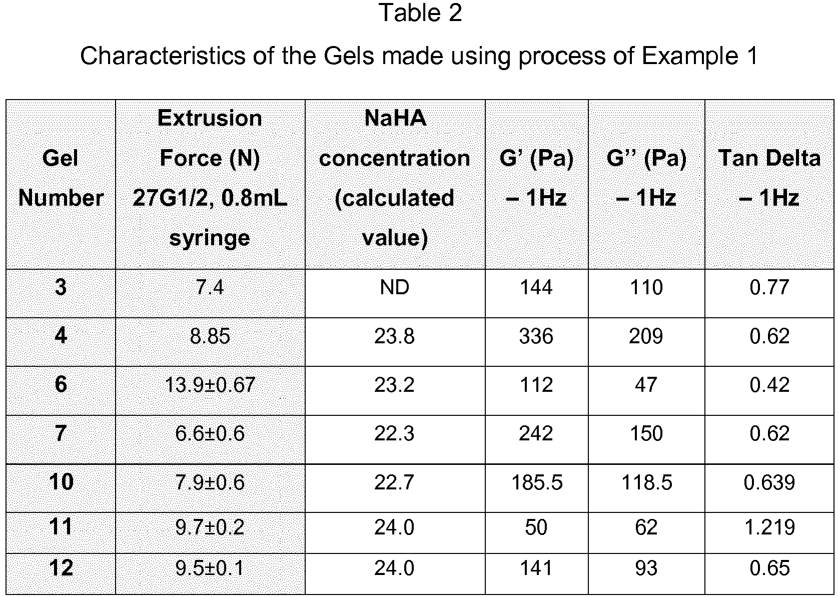

### Visual demonstration of cohesivity

While the compression test run with a rheometer is quantitative, the gel is compressed at a constant speed but cannot be seen. Another way to run a compression more visually is to compress the gels at constant weight, until an equilibrium is reached when the gel opposes a force which is enough to sustain the weight above it.

Standard microscope slides (about 100 mm x 25 mm, and 0.1mm thickness) are used to press on 0.1mL of a hydrogel and the shape of the gel can be followed since the slides are transparent glass. The more cohesive the gel is with this set-up, the thicker the gel will remain when compressed by a glass slide. Several glass slides can be added to modify the weight. Gel formulations can be ranked by final gel thickness when the equilibrium is reached.

It should be noted that this test only works with HA-based hydrogels. If the formulation contains solid particles, cohesivity may be overestimated.

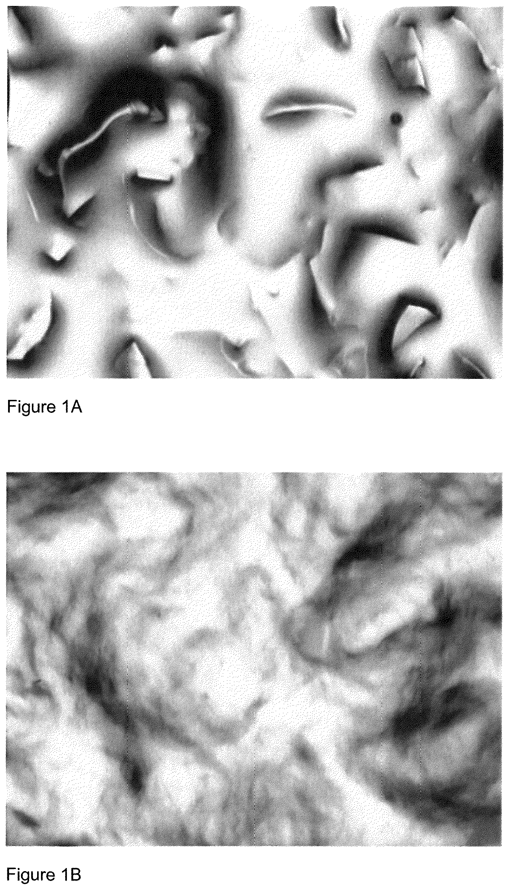

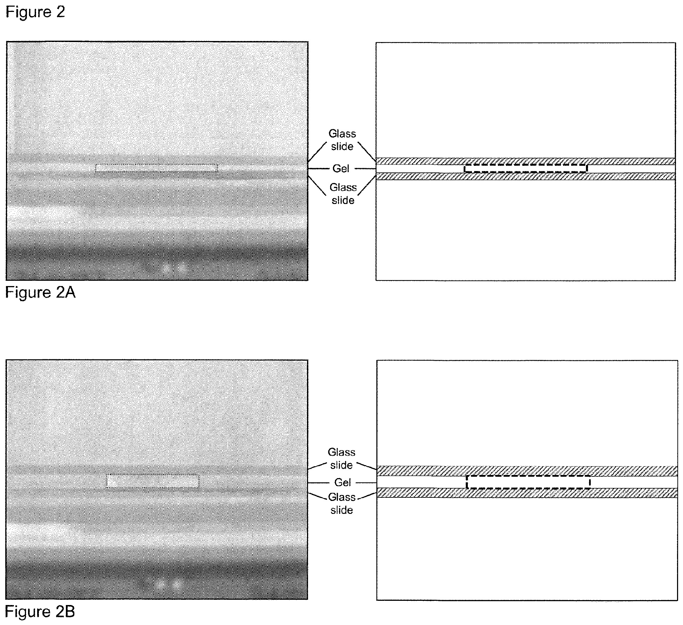

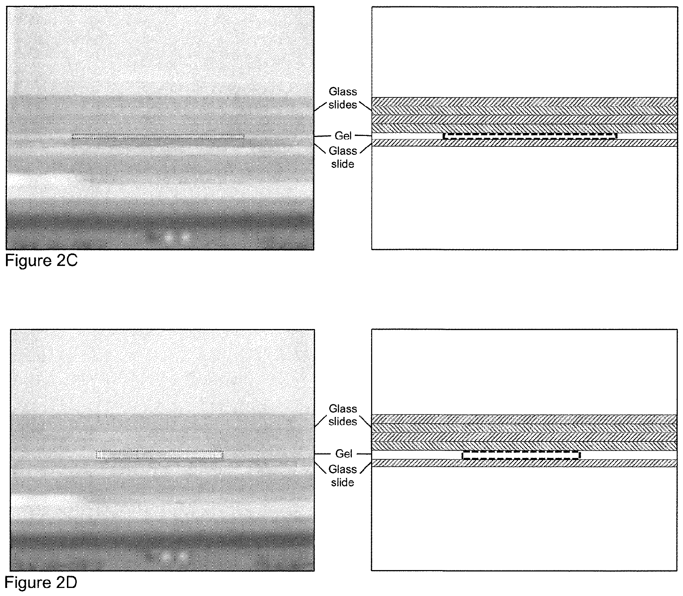

Figure 2 illustrates this effect. The differences between a gel made of a suspension of particles (Restylane- SubQ, Figs. 2A and 2C, very low cohesivity, below 20 gmf) and a monophasic gel with high cohesivity (Figs 2B and 2D, above 60 gmf). Pictures show the equilibrium state with one glass slide on the gels (Figures 2A and 2B) and four glass slides on the gels (Figures 2C and 2D). The schematic drawings supplementing the pictures further illustrate the observed effect. One glass slide was used to support the gel in all experiments. Compression of the gels was accomplished with one (Figures 2A and 2B) or four (Figures 2C and D) glass slides, respectively. The compressed gels in the pictures and the drawings are outlined with a dashed line for better comparison of their thickness. The tests demonstrate that the highly cohesive gel resists compression better than the low cohesivity gel.

A range of different crosslinked gels could be prepared using the methods of the present invention, the gels having a range of broad properties. It has been discovered that the gel elastic and viscous modulus increases with TMP content until TMP solubility prevents further crosslinking (generally, above 300 wt%) and yields fragmented gels with hard domains and non-crosslinked domains.

A general characteristic of the present TMP-based gels is a very high viscous modulus providing good flowing properties although the elastic modulus shown in Table 2 are still within in a range which allows their use as dermal fillers.

## Claims

1. A method of making a monophasic and cohesive dermal filler comprising a hyaluronic acid (HA) crosslinked with a trimetaphosphate (TMP), wherein the method comprises the steps of:
- Mixing at least one trimetaphosphate salt with at least one hyaluronan (HA), with a TMP to HA weight ratio comprised between 1:1 and 3:1, and preferably comprised between 1.5:1 and 2:1;
- Dissolving the TMP-HA mix in an aqueous solution with a pH at least 9, preferably at least 11 and most preferably at least 11, so that the HA concentration is comprised between 80 and 100 mg/mL, and preferably about 90 mg/mL;
- Heating the mixture between 20°C and 70°C for 30 min to 72 hours, causing the HA to become crosslinked with the trimetaphosphate salt to form a monophasic and cohesive gel;
- Removing the unreacted TMP and excess of salts;
- Homogeneizing and diluting the gel to its final HA concentration;
- Filling into syringes; and
- Sterilizing the hydrogel by autoclaving and/or electron-beam.

2. The method of claim 1, wherein the TMP comprises a salt of trimetaphosphate selected from the group consisting of sodium trimetaphosphate, calcium trimetaphosphate and barium trimetaphosphate.

3. The method of claim 1, wherein the final HA concentration is comprised between 5 and 40 mg/g, preferably between 10 and 30 mg/g, and most preferably between 15 and 25 mg/g.

4. The method of claim 1, wherein the HA used for crosslinking comprises at least 80 wt%, preferably at least 90 wt%, most preferably 100 wt% of a HA having a molecular weight of at least 2,000,000 Da or an intrinsic viscosity of at least 2.2 L/g.

5. The method of any one of claims 1 to 4, wherein the dermal filler has a compression force (Fn) of at least 0.20 N (20 gmf), preferably at least 0.39 N (40 gmf), most preferably at least 0.59 N (60 gmf).

6. The method of any one of claims 1 to 4, wherein an uncrosslinked HA fraction is added at any step after crosslinking and is present at a concentration between 0.1 and 3 mg/g, and preferably between 0.2 and 1.5 mg/g in the final composition

7. The method of any one of claims 1 to 4, wherein at least one uncrosslinked or crosslinked glycosaminoglycan (GAG) is added at any step after crosslinking and is present at a concentration between 1 and 40 mg/g

8. The method of any one of claims 1 to 4, wherein at least one anesthetic agent, preferably a local anesthetic agent is added at any step after crosslinking and is present at a weight percent of the total composition between 0.1 and 5 wt%.

9. The method of any one of claims 1 to 4, wherein at least one anti-oxidant agent is added at any step after crosslinking and is present at a weight percent of the total composition between 0.001 and 10 wt%.

## Patentansprüche

1. Verfahren zur Herstellung eines einphasigen und kohäsiven dermalen Füllstoffs, der eine Hyaluronsäure (HA), die mit einem Trimetaphosphat (TMP) vernetzt ist, umfasst, wobei das Verfahren die folgenden Schritte umfasst:
- mindestens ein Trimetaphosphatsalz wird mit mindestens einer Hyaluronsäure (HA) gemischt, wobei das TMP zu HA Gewichtsverhältnis zwischen 1:1 und 3:1 liegt und vorzugsweise zwischen 1,5:1 und 2:1 liegt;
- die TMP-HA-Mischung wird in einer wässrigen Lösung mit einem pH-Wert von mindestens 9, vorzugsweise mindestens 11 und am meisten bevorzugt mindestens 11 so gelöst, dass die HA Konzentration zwischen 80 und 100 mg/mL liegt und vorzugsweise ungefähr 90 mg/mL beträgt;
- die Mischung wird zwischen 20 °C und 70 °C für 30 Minuten bis 72 Stunden erwärmt, wodurch die HA mit dem Trimetaphosphatsalz so vernetzt wird, dass ein einphasiges und kohäsives Gel gebildet wird;
- das nicht umgesetzte TMP und überschüssige Salze werden entfernt;
- das Gel wird homogenisiert und so verdünnt, dass es seine endgültige HA Konzentration erreicht;
- Abfüllen in Spritzen; und
- das Hydrogel wird durch Autoklavieren und/oder Elektronenstrahl-Behandlung sterilisiert.

2. Verfahren nach Anspruch 1, bei dem das TMP ein Trimetaphosphatsalz ausgewählt aus der Gruppe bestehend aus Natriumtrimetaphosphat, Calciumtrimetaphosphat und Bariumtrimetaphosphat umfasst.

3. Verfahren nach Anspruch 1, bei dem die endgültige HA Konzentration zwischen 5 und 40 mg/g, vorzugsweise zwischen 10 und 30 mg/g und am meisten bevorzugt zwischen 15 und 25 mg/g liegt.

4. Verfahren nach Anspruch 1, bei dem die HA, die für das Vernetzen verwendet wird, mindestens 80 Gewichts-%, vorzugsweise mindestens 90 Gewichts-% und am meisten bevorzugt 100 Gewichts-% einer HA mit einem Molekulargewicht von mindestens 2.000.000 Da oder einer intrinsischen Viskosität von mindestens 2,2 L/g umfasst.

5. Verfahren nach mindestens einem der Ansprüche 1 bis 4, bei dem der dermale Füllstoff eine Kompressionskraft (Fn) von mindestens 0,20 N (20 gmf), vorzugsweise mindestens 0,39 N (40 gmf) und am meisten bevorzugt mindestens 0,59 N (60 gmf) aufweist.

6. Verfahren nach mindestens einem der Ansprüche 1 bis 4, bei dem eine nicht-vernetzte HA-Fraktion bei einem beliebigen Schritt nach dem Vernetzen hinzugefügt wird und in einer Konzentration zwischen 0,1 und 3 mg/g und vorzugsweise zwischen 0,2 und 1,5 mg/g in der endgültigen Zusammensetzung vorhanden ist.

7. Verfahren nach mindestens einem der Ansprüche 1 bis 4, bei dem mindestens ein nicht-vernetztes oder vernetztes Glykosaminglykan (GAG) bei einem beliebigen Schritt nach dem Vernetzen hinzugefügt wird und in einer Konzentration zwischen 1 und 40 mg/g vorhanden ist.

8. Verfahren nach mindestens einem der Ansprüche 1 bis 4, bei dem mindestens ein Anästhesiemittel, vorzugsweise ein lokales Anästhesiemittel, bei einem beliebigen Schritt nach dem Vernetzen hinzugefügt wird und zu einem Gewichtsprozentsatz der gesamten Zusammensetzung zwischen 0,1 und 5 Gewichts-% vorhanden ist.

9. Verfahren nach mindestens einem der Ansprüche 1 bis 4, bei dem mindestens ein Antioxidans bei einem beliebigen Schritt nach dem Vernetzen hinzugefügt wird und zu einem Gewichtsprozentsatz der gesamten Zusammensetzung zwischen 0,001 und 10 Gewichts-% vorhanden ist.

## Revendications

1. Procédé de préparation d'une charge dermique monophasique et cohésive comprenant un acide hyaluronique (HA) réticulé avec un trimétaphosphate (TMP), dans lequel le procédé comprend les étapes suivantes :
- mélange d'au moins un sel de trimétaphosphate avec au moins un hyaluronane (HA), avec un rapport en poids TMP:HA compris entre 1:1 et 3:1, et de préférence compris entre 1,5:1 et 2:1 ;
- dissolution du mélange TMP-HA dans une solution aqueuse avec un pH d'au moins 9, de préférence d'au moins 11 et le plus de préférence d'au moins 11, de telle sorte que la concentration en HA est comprise entre 80 et 100 mg/ml, et de préférence d'environ 90 mg/ml ;
- chauffage du mélange entre 20 °C et 70 °C pendant 30 minutes à 72 heures, provoquant la réticulation du HA avec le sel de trimétaphosphate pour former un gel monophasique et cohésif ;
- élimination du TMP n'ayant pas réagi et de l'excès de sels ;
- homogénéisation et dilution du gel à sa concentration finale en HA ;
- remplissage dans des seringues ; et
- stérilisation de l'hydrogel par autoclavage et/ou faisceau d'électrons.

2. Procédé selon la revendication 1, dans lequel le TMP comprend un sel de trimétaphosphate sélectionné à partir du groupe constitué par le trimétaphosphate de sodium, le trimétaphosphate de calcium et le trimétaphosphate de baryum.

3. Procédé selon la revendication 1, dans lequel la concentration finale en HA est comprise entre 5 et 40 mg/g, de préférence entre 10 et 30 mg/g, et le plus de préférence entre 15 et 25 mg/g.

4. Procédé selon la revendication 1, dans lequel le HA utilisé pour la réticulation comprend au moins 80 % en poids, de préférence au moins 90 % en poids, le plus de préférence au moins 100 % en poids d'un HA ayant un poids moléculaire d'au moins 2 000 000 Da ou une viscosité intrinsèque d'au moins 2,2 l/g.

5. Procédé selon l'une quelconque des revendications 1 à 4, dans lequel la charge dermique a une force de compression (Fn) d'au moins 0,20 N (20 gmf), de préférence d'au moins 0,39 N (40 gmf), le plus de préférence d'au moins 0,59 N (60 gmf).

6. Procédé selon l'une quelconque des revendications 1 à 4, dans lequel une fraction de HA non réticulé est ajoutée à toute étape après la réticulation et est présente en une concentration comprise entre 0,1 et 3 mg/g, et de préférence entre 0,2 et 1,5 mg/g dans la composition finale.

7. Procédé selon l'une quelconque des revendications 1 à 4, dans lequel au moins un glycosaminoglycane (GAG) non réticulé ou réticulé est ajouté à toute étape après la réticulation et est présent en une concentration comprise entre 1 et 40 mg/g.

8. Procédé selon l'une quelconque des revendications 1 à 4, dans lequel au moins un agent anesthésique, de préférence un agent anesthésique local est ajouté à toute étape après la réticulation et est présent en un pourcentage en poids de la composition totale entre 0,1 et 5 % en poids.

9. Procédé selon l'une quelconque des revendications 1 à 4, dans lequel au moins un agent antioxydant est ajouté à toute étape après la réticulation et est présent en un pourcentage en poids de la composition totale entre 0,001 et 10 % en poids.
